# EUROPEAN PATENT APPLICATION

(11) **EP 1 759 685 A1**
(43) Date of publication of application: **07.03.2007**
(21) Application number: 05019074.3
(22) Date of filing: 02.09.2005
(51) Int. Cl.: A61K 8/23

(54) **Agent for simultaneous lightening and coloring of keratin fibres containing a sulfamate bleach booster**

(71) Applicant: Wella Aktiengesellschaft, 64274 Darmstadt (DE)
(72) Inventor: Javet, Manuela, 1723 Marly (CH); Müller, Catherine, 1723 Marly (CH); Weber, Ingo, 1723 Marly (CH)

(57) **Abstract**

The present patent application relates to a ready-to-use agent for simultaneously lightening and dyeing keratin fibers (especially human hair), having a basic pH and being characterized by containing a) at least one bleach booster according to the general formula (I) 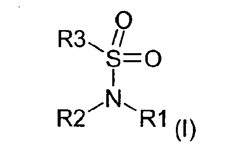 wherein **R1** is a hydrogen, **R2** is hydrogen, a substituted or unsubstituted C1- to C12-alkyl group, a substituted or unsubstituted C1- to C12-monohydroxy-alkyl group, a substituted or unsubstituted C2- to C12-polyhydroxy alkyl group, or a substituted saturated, unsaturated or aromatic 4- to 8-membered carbocycle or heterocycle, and **R3** is a OR-Group, with **R** being equal to hydrogen, an ammonium group or an alkali metal or an earth alkali metal atom; and
b) at least one direct dye and/or at least one oxidation dye/dye precursor and c) at least one oxidant;

a multi-component-kit and a method for simultaneous lightening and coloring hair.

## Description

The present invention relates to hair bleaching agents capable of lightening natural fibers up to four or five levels and simultaneously coloring fibers (e. g. wool, fur, or hair, especially human hair) with direct dyes or/and oxidative dyes (dye precursors), in a single step.

Natural hair color is based on melanin granules embedded throughout the cortex of hair fibers. Two general classes of such pigments have been identified: eumelanins (brownish black) and pheomelanins (reddish orange). The combination ratio and concentration of these two types of pigments impart to the hair its characteristic natural gradation of color. Dark hair has a higher concentration of the eumelanins, while red hair has a predominance of the pheomelanins light hair has reduced amounts of both.

Human hair color is arbitrarily classified in a scale of levels describing its darkness (or lightness). Black hair is designated as level one, medium brown hair as level five, and pale light blond hair as level ten, with several nuances in-between.

Hair bleaching is a chemical process by which the melanin pigment granules are gradually destroyed by the use of a so-called bleaching agent, resulting in a lighter color. Bleaching is complex, because it calls on a range of chemical reactions involving not only the pigment it is intended to lighten, but also to the keratin fiber itself. It is not yet possible to act on melanin without using reagents that modify polypeptide (side) chains and the network of linkages binding them together.

Bleaching is important because it concerns more than just the subject of the decolorizing of hair; it is involved in the majority of all coloring operations. Fine coloring, which is transparent and subtle, cannot exist without simultaneous bleaching, and as mentioned previously, all coloring systems based on oxidation dyes use bleaching to some extent. Bleaching is therefore one of the most important hair treatment operations. Based on their chemical compositions and strength, hair bleaches may be classified in two categories:
1. Type-1 Bleaches: Relatively mild liquid or cream-based compositions utilizing ammonia/hydrogen peroxide solutions as the main oxygen-generating agent to oxidize and bleach the melanin, usually in conjunction with a hair coloring process. Just before use, the peroxide is mixed with the alkalizer, and the resulting liquid or creme is applied to hair for 30 to 60 minutes. Such compositions may lighten the hair by as much as four levels at the most, depending on the concentration of hydrogen peroxide used. The lightening of one to two levels is the most common.
2. Type-2 Bleaches: These are mostly powder compositions containing persulfate salts (ammonium, potassium, sodium) as bleach booster supplies, which are mixed with hydrogen peroxide just before use and applied on the hair for 30 to 50 minutes. These compositions may lighten the hair by over seven levels.

The type-1 bleaches constitute most of what is known as the current high-lifting shades of commercial permanent hair colorants. These colorants are often based on oxidative primary intermediates and couplers, and can contain direct dyes, disperse dyes, acid dyes, or basic dyes. The used alkaline peroxide environment is mild enough to allow the survival of several types of dyes.

In the type-2 bleaches, the medium is quite hostile to most dyes. The higher alkalinity and stronger oxidizing conditions act synergistically to destroy the present dyes within a short period of time. Therefore only few dyes can be used in combination with these bleaches.

There are many publications and patent applications or patents dealing with the art of simultaneous bleaching and coloring of hair, for example K. Schrader "Grundlagen und Rezepturen der Kosmetika", 2^{nd} edition (1989), page 783 ff, as well as US 5688291 A, US 3578387 A, US 3912446 A, DE 19961273 U1 and WO 97/39727 A1.

Normally, a type-1 bleach with simultaneous dyeing only allows a relatively small variation of the hair color because of the remaining undestroyed melanin. In addition common hair dyes don't survive the type-2 bleach process. In addition, type-2 bleaches show a lot of fiber damage and often a undesirable orange hue.

Surprisingly, we found bleach boosters which allow simultaneously dyeing and bleaching of fibers with a lightening in-between the type-1 bleaches and the type-2 bleaches. In addition to the higher bleaching levels, we observe are more natural, less orange hue in lighter shades due to the more efficient degradation of melanin. The fiber damage is comparable to a type-1 bleach.

It is known from DE 19961273 A1 to use special sulfamates as bleach booster.

Hair colorants are primarily divided into oxidation colorants and tints depending on the starting hair color to be colored and the desired end result. Oxidation hair colors are exceptionally suitable for covering relatively large amounts of gray, with the oxidation colorants used for a gray content of up to 50% generally being referred to as oxidative tints, while the oxidation colorants used for a gray content of more than 50% or for "lightening" are generally referred to as so-called oxidative colors. Direct dyes are primarily present in non-oxidative colorants (so-called tinting agents). Some direct dyes, such as, for example nitro dyes, can, on account of their small size, penetrate into the hair and color it directly - at least in the outer regions. Such tints are very gentle on the hair and generally withstand 6 to 8 hair washes. Direct dyes are likewise often used in oxidative colorants for producing certain shades or for intensifying the color.

The object of the present invention is a ready-to-use agent for simultaneously lightening and dyeing keratin fibers (especially human hair), having a basic pH and being characterized by containing
a) at least one bleach booster according to the general formula (I) 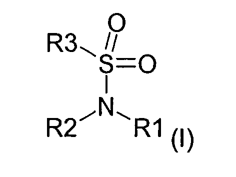 wherein **R1** is a hydrogen, **R2** is hydrogen, a substituted or unsubstituted C1- to C12-alkyl group, a substituted or unsubstituted C1- to C12-monohydroxy-alkyl group, a substituted or unsubstituted C2- to C12-polyhydroxy alkyl group, or a substituted saturated, unsaturated or aromatic 4- to 8-membered carbocycle or heterocycle, and **R3** is a OR-Group, with **R** being equal to hydrogen, an ammonium group or an alkali metal or an earth alkali metal atom; and
b) at least one direct dye and/or at least one oxidation dye/dye precursor and c) at least one oxidant.

The bleach booster of the general formula (I) is preferably selected from among sodium cyclohexanesulfamate; ammonium cyclohexanesulfamate; potassium cyclohexanesulfamate; cyclohexane sulfamic acid (cyclamate); sulfamic acid; sodium sulfamate; potassium sulfamate; ammonium sulfamate; sodium phenylsulfamate; ammonium phenylsulfamate; potassium phenylsulfamate; phenyl sulfamic acid; sodium benzylsulfamate; ammonium benzylsulfamate; potassium benzylsulfamate; benzyl sulfamic acid; sodium isopropylsulfamate; ammonium isopropylsulfamate; potassium isopropylsulfamate; isopropyl sulfamic acid; sodium methylsulfamate; ammonium methylsulfamate; potassium methylsulfamate; methyl sulfamic acid; sodium t-butylsulfamate; ammonium t-butylsulfamate; potassium t-butylsulfamate and t-butyl sulfamic acid.

The bleach booster is contained in the ready-to-use agent in an amount from about 0.01 to 70 weight percent and preferably from about 1 to 50 weight percent.

Preferably the following oxidative dyes/dye precursors may be used: 1,4-diaminobenzene (p-phenylenediamine), 1,4-diamino-2-methylbenzene (p-toluylenediamine), 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 2-(6-2,5-methyl-pyridin-2-yl)-benzene-1,4-diamine, 2-thiazol-2-yl-benzene-1,4-diamino, 1,4-diamino-2-(pyridin-3-yl)benzene, 2,5-diaminobiphenyl, 2,5-diamino-4'-(1-methylethyl)-1,1'-biphenyl, 2,3',5-triamino-1,1'-biphenyl, 2'-chloro-1,1'-biphenyl-2,5-diamine, 3'-fluoro-1,1'-biphenyl-2,5-diamine, 1,4-diamino-2-methoxymethylbenzene, 1,4-diamino-2-aminomethyl-benzene, 3-(3-amino-phenylamino-propenyl)-benzene-1,4-diamine, 2-propenylbenzene-1,4-diamine, 1,4-diamino-2-((phenylamino)methyl)-benzene, 1,4-diamino-2-((ethyl-(2-hydroxyethyl)-amino)methyl)benzene, 1,4-diamino-2-hydroxymethylbenzene, 1,4-diamino-2-(2-hydroxyethoxy)-benzene, 2-(2-(acetylamino)ethoxy)-1,4-diaminobenzene, 4-(phenyl-amino)aniline, 4-(dimethylamino)aniline, 4-(diethylamino)aniline, 4-(dipropylamino)aniline, 4-[ethyl(2-hydroxyethyl)amino]aniline, 4-[di(2-hydroxyethyl)amino]aniline, 4-[di(2-hydroxyethyl)amino]-2-methyl-aniline, 4-[(2-methoxyethyl)amino]aniline, 4-[(3-hydroxypropyl)amino]aniline, 4-[(2,3-dihydroxypropyl)amino]aniline, 4-(((4-aminophenyl)methyl)-amino)aniline, 4-[(4-amino-phenylamino)-methyl]-phenol, 3-((4-amino-phenylamino)methyl)phenol, 1,4-diamino-n-(4-pyrrolidin-1-yl-benzyl)-benzene, 1,4-diamino-n-furan-3-ylmethylbenzene, 1,4-diamino-n-thiophen-2-ylmethylbenzene, 1,4-diamino-n-furan-2-ylmethylbenzene, 1,4-diamino-n-thiophen-3-ylmethylbenzene, 1,4-diamino-n-benzylbenzene, 1,4-diamino-2-(1-hydroxyethyl)benzene, 1,4-diamino-2-(2-hydroxyethyl)-benzene, 1,4-diamino-2-(1-methylethyl)benzene, 1,3-bis[(4-aminophenyl)-(2-hydroxyethyl)amino]-2-propanol, 1,4-bis[(4-aminophenyl)amino]-butane, 1,8-bis(2,5-diaminophenoxy)-3,6-dioxaoctane, 2,5-diamino-4'-hydroxy-1,1'-biphenyl, 2,5-diamino-2'-trifluoromethyl-1,1'-biphenyl, 2,4',5-triamino-1,1'-biphenyl, 4-amino-phenol, 4-amino-3-methylphenol, 4-amino-3-(hydroxymethyl)-phenol, 4-amino-3-fluoro-phenol, 4-methylamino-phenol, 4-amino-2-(aminomethyl)phenol, 4-amino-2-(hydroxymethyl)phenol, 4-amino-2-fluorophenol, 4-amino-2-[(2-hydroxyethyl)-amino]methyl-phenol, 4-amino-2-methyl-phenol, 4-amino-2-(methoxymethyl)phenol, 4-amino-2-(2-hydroxyethyl)phenol, bis(5-amino-2-hydroxyphenyl)phenol, 5-amino-salicylic acid, 2,5-diamino-pyridine, 2,4,5,6-tetraamino-pyrimidine, 2,5,6-triamino-4-(1h)-pyrimidone, 4,5-diamino-1-(2-hydroxyethyl)-1h-pyrazole, 4,5-diamino-1-(-methylethyl)-1h-pyrazole, 4,5-diamino-1-[(4-methylphenyl)methyl]-1h-pyrazole, 1-[(4-chlorophenyl)methyl]-4,5-diamino-1 h-pyrazole, 4,5-diamino-1-methyl-1h-pyrazole, 4,5-diamino-1-pentyl-1h-pyrazole, 4,5-diamino-1-(phenylmethyl)-1h-pyrazole, 4,5-diamino-1-((4-methoxyphenyl)methyl-1 h-pyrazole, 2-aminophenol, 2-amino-6-methylphenol, 2-amino-5-methyl-phenol, 4-amino-1,1'-biphenyl-3-ol, 2-amino-5-ethylphenol, 1,2,4-trihydroxybenzene, 2,4-diaminophenol, 1,4-dihydroxybenzene, 2-(((4-aminophenyl)amino)methyl)-1,4-diaminobenzene;
n-((3-dimethylamino)phenyl)urea, 2,6-diaminopyridine, 2-amino-4-[(2-hydroxyethyl)amino]-anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)-amino]aniline, 1,3-di(2,4-diaminophenoxy)propane, di(2,4-diamino-phenoxy)methane, 1,3-diamino-2,4-dimethoxybenzene, 2,6-bis(2-hydroxyethyl)amino-toluene, 4-hydroxyindole, 3-dimethylaminophenol, 3-(diethylamino)phenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-amino-phenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino)-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol-acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 6-hydroxy-benzomorpholine, 6-aminobenzomorpholine, 3-methyl-1-phenyl-5-pyrazolone, 5,6-dihydroxyindole, 5,6-dihydroxyindoline, 5-hydroxyindole, 6-hydroxyindole, 7-hydroxyindole, 2,3-indolinedione.

Preferably the following direct dyes may be used:
1,4-bis[(2-hydroxyethyl)amino]-2-nitrobenzene, 1-(2-hydroxyethyl)amino-2-nitro-4-[di(2-hydroxyethyl)amino]-benzene, (HC blue no. 2), 1-amino-3-methyl-4-[(2-hydroxyethyl)amino]-6-nitrobenzene, (HC violet no. 1), 4-[ethyl-(2-hydroxyethyl)amino]-1-[(2-hyd roxyethyl)amino]-2-nitrobenzene-hydrochloride (HC blue no. 12), 4-[di(2-hydroxyethyl)amino]-1-[(2-methoxyethyl)amino]-2-nitrobenzene (HC blue no. 11), 1-[(2,3-dihydroxypropyl)amino]-4-[methyl-(2-hydroxyethyl)amino]-2-nitrobenzene (HC blue no. 10), 1-[(2,3-dihydroxypropyl)amino]-4-[ethyl-(2-hydroxyethyl)amino]-2-nitrobenzene-hydrochloride (HC blue no. 9), 1-(3-hydroxypropylamino)-4-[di(2-hydroxyethyl)amino]-2-nitrobenzene (HC violet no. 2), 1-methylamino-4-[methyl-(2,3-dihydroxypropyl)amino]-2-nitrobenzene (HC blue no. 6), 2-((4-amino-2-nitrophenyl)amino)-5-dimethylaminobenzoic acid (HC blue no. 13), 1-(2-aminoethylamino)-4-[di(2-hydroxyethyl)amino]-2-nitrobenzene, 4-(di(2-hydroxyethyl)amino)-2-nitro-1-phenylamino-benzene, 1-amino-4-[(2-hydroxyethyl)amino]-2-nitrobenzene (HC red no. 7), 2-amino-4,6-dinitro-phenol, 1,4-diamino-2-nitrobenzene (CI 76070), 4-amino-2-nitro-diphenylamine (HC red no. 1), 1-amino-4-[di(2-hydroxyethyl)amino]-2-nitrobenzene-hydrochloride (HC red no. 13), 1-amino-5-chloro-4-[(2-hydroxyethyl)amino]-2-nitrobenzene, 4-amino-1-[(2-hydroxyethyl)amino]-2-nitrobenzene (HC red no. 3), 4-((2-hydroxyethyl)methylamino)-1-(methylamino)-2-nitrobenzene, 1-amino-4-((2,3-dihydroxypropyl)amino)-5-methyl-2-nitrobenzene, 1-amino-4-(methylamino)-2-nitrobenzene, 4-amino-2-nitro-1-((prop-2-en-1-yl)amino)-benzene, 4-amino-3-nitrophenol, 4-[(2-hydroxyethyl)amino]-3-nitrophenol, 4-[(2-nitrophenyl)amino]phenol (HC orange no. 1), 1-[(2-aminoethyl)amino]-4-(2-hydroxyethoxy)-2-nitrobenzene (HC orange no. 2), 4-(2,3-dihydroxypropoxy)-1-[(2-hydroxyethyl)amino]-2-nitrobenzene (HC orange no. 3), 1-amino-5-chloro-4-[(2,3-dihydroxypropyl)amino]-2-nitrobenzene (HC red no. 10), 5-chloro-1,4-[di(2,3-dihydroxypropyl)amino] -2-nitrobenzene (HC red no. 11), 2-[(2-hydroxyethyl)amino]-4,6-dinitrophenol, 4-ethylamino-3-nitrobenzoic acid, 2-[(4-amino-2-nitrophenyl)amino]-benzoic acid, 2-chloro-6-ethylamino-4-nitrophenol, 2-amino-6-chloro-4-nitrophenol, 4-[(3-hydroxypropyl)amino]-3-nitrophenol, 2,5-diamino-6-nitropyridine, 6-amino-3-((2-hydroxyethyl)amino)-2-nitropyridine, 3-amino-6-((2-hydroxyethyl)amino)-2-nitropyridine, 3-amino-6-(ethylamino)-2-nitropyridine, 3-((2-hydroxyethyl)amino)-6-(methylamino)-2-nitropyridin, 3-amino-6-(methylamino)-2-nitropyridine, 6-(ethylamino)-3-((2-hydroxyethyl)amino)-2-nitropyridine, 1,2,3,4-tetrahydro-6-nitroquinoxaline, 7-amino-3,4-dihydro-6-nitro-2h-1,4-benzoxazine (HC red no. 14), 1,2-diamino-4-nitrobenzene (CI 76020), 1-amino-2-[(2-hydroxyethyl)amino]-5-nitrobenzene (HC yellow no. 5), 1-(2-hydroxy-ethoxy)-2-[(2-hydroxyethyl)amino]-5-nitrobenzene (HC yellow no. 4), 1-[(2-hydroxyethyl)amino]-2-nitrobenzene (HC yellow no. 2), 2-(di(2-hydroxyethyl)amino)-5-nitrophenol, 2-[(2-hydroxyethyl)amino]-1-methoxy-5-nitrobenzene, 2-amino-3-nitrophenol, 1-amino-2-methyl-6-nitrobenzene, 1-(2-hydroxyethoxy)-3-methylamino-4-nitrobenzene, 2,3-(dihydroxypropoxy)-3-methylamino-4-nitrobenzene, 2-[(2-hydroxyethyl)amino]-5-nitrophenol (HC yellow no. 11), 3-[(2-aminoethyl)amino]-1-methoxy-4-nitrobenzene-hydrochloride (HC yellow no.9), 1-[(2-ureidoethyl)amino]-4-nitrobenzene, 4-[(2,3-dihydroxypropyl)amino]-3-nitro-1-trifluoromethyl-benzene (HC yellow no. 6), 1-chloro-2,4-bis[(2-hydroxyethyl)amino]-5-nitrobenzene (HC yellow no. 10), 1-amino-4-((2-aminoethyl)amino)-5-methyl-2-nitrobenzene, 4-[(2-hydroxyethyl)amino]-3-nitro-1-methylbenzene, 1-chloro-4-[(2-hydroxyethyl)amino]-3-nitrobenzene (HC yellow no. 12), 4-[(2-hydroxyethyl)amino]-3-nitro-1-trifluoromethylbenzene (HC yellow no. 13), 4-[(2-hydroxyethyl)amino]-3-nitro-benzonitrile (HC yellow no. 14), 4-[(2-hydroxyethyl)amino]-3-nitro-benzamide (HC yellow no. 15), 3-((2-hydroxyethyl)amino)-4-methyl-1-nitrobenzene, 4-chloro-3-((2-hydroxyethyl)amino)-1-nitrobenzene, 2,4-dinitro-1-hydroxy-naphthaline, 1,4-di[(2,3-dihydroxypropyl)amino]-9,10-anthraquinone, 1,4-di[(2-hydroxyethyl)amino]-9,10-anthraquinone (Cl 61545, disperse blue no. 23), disperse blue no. 377, 1-[(2-hydroxyethyl)amino]-4-methylamino-9,1 0-anthraquinone (CI 61505, disperse blue no. 3), 2-[(2-aminoethyl)amino]-9,10-anthraquinone (HC orange no. 5), 1-amino-4-hydroxy-9,10-anthraquinone (Cl 60710, disperse red 15), 1-hydroxy-4-[(4-methyl-2-sulfophenyl)amino]-9,10-anthraquinone, 7-beta-d-glucopyranosyl-9,10-dihydro-1-methyl-9,10-dioxo-3,5,6,8-tetrahydroxy-2-anthracencarboxylic acid (Cl 75470, natural red no. 4), 1-[(3-aminopropyl)amino]-4-methylamino-9,10-anthraquinone (HC blue no. 8), 1-[(3-aminopropyl)-amino]-9,10-anthraquinone (HC red no. 8), 1,4-diamino-2-methoxy-9,10-anthraquinone (Cl 62015, disperse red no. 11, solvent violet no. 26), 1,4-dihydroxy-5,8-bis[(2-hydroxyethyl)amino]-9,10-anthraquinone (Cl 62500, disperse blue no. 7, solvent blue no. 69), 1,4-diamino-9,10-anthraquinone (Cl 61100, disperse violet no. 1), 1-amino-4-(methylamino)-9,10-anthraquinone (Cl 61105, disperse violet no. 4, solvent violet no.12), 2-hydroxy-3-methoxy-1,4-naphthoquinone, 2,5-dihydroxy-1,4-naphthoquinone, 2-hydroxy-3-methyl-1,4-naphthoquinone, n-(6-((3-chloro-4-(methylamino)phenyl)imino)-4-methyl-3-oxo-1,4-cyclohexadien-1-yl)urea (HC red no. 9), 2-((4-(di(2-hydroxyethyl)amino)phenyl)amino)-5-((2-hydroxyethyl)amino)-2,5-cyclohexadien-1,4-dione (hc green no. 1), 5-hydroxy-1,4-naphthoquinone (Cl 75500, natural brown no. 7), 2-hydroxy-1,4-naphthoquinone (Cl 75480, natural orange no. 6), 1,2-dihydro-2-(1,3-dihydro-3-oxo-2h-indol-2-yliden)-3h-indol-3-on (CI 73000), 1,3-bis(dicyanomethylen)indan, 9-(dimethylamino)-benzo[a]phenoxazin-7-ium-chloride (Cl 51175; basic blue no. 6), di[4-(diethylamino)phenyl][4-(ethylamino)naphthyl]carbenium-chloride (Cl 42595; basic blue no. 7), di-(4-(dimethylamino)phenyl)-(4-(methyl-phenylamino)naphthalin-1-yl)carbenium-chloride (Cl42563; basic blue no. 8), 3,7-di(dimethylamino)phenothiazin-5-ium-chloride (Cl 52015; basic blue no. 9), di[4-(dimethylamino)phenyl][4-(phenylamino)naphthyl]carbenium-chloride (CI 44045; basic blue no. 26), 2-[(4-(ethyl(2-hydroxyethyl)amino)-phenyl)azo]-6-methoxy-3-methyl-benzothiazolium-methylsulfate (CI 11154; basic blue no. 41), basic blue no. 77, 8-amino-2-bromo-5-hydroxy-4-imino-6-[(3-(trimethylammonio)phenyl)amino]-1(4h)-naphthalinone-chloride (CI 56059; basic blue no. 99), bis[4-(dimethylamino)phenyl][4-(methylamino)phenyl]carbenium-chloride (Cl 42535; basic violet no. 1), tri(4-amino-3-methylphenyl)carbenium-chloride (CI 42520; basic violet no. 2), tris[4-(dimethylamino)phenyl]carbenium-chloride (CI42555; basic violet no. 3), 2-[3,6-(diethylamino)dibenzopyranium-9-yl]-benzoic acide chloride (Cl 45170; basic violet no. 10), di(4-aminophenyl)(4-amino-3-methylphenyl)carbenium-chloride (Cl 42510; basic violet no. 14), 1 ,3-bis[(2,4-diamino-5-methylphenyl)azo]-3-methylbenzene (Cl 21010; basic brown no. 4), 1-[(4-aminophenyl)azo]-7-(trimethylammonio)-2-naphthol-chloride (Cl 12250; basic brown no. 16), 3-[(4-amino-2,5-dimethoxyphenyl)azo]-n,n,n-trimethylbenzeneaminium-chloride . (Cl 112605, basic orange no. 69), 1-[(4-amino-2-nitrophenyl)azo]-7-(trimethylammonio)-2-naphthol-chloride (basic brown no. 17), 1-[(4-amino-3-nitrophenyl)azo]-7-(trimethylammonio)-2-naphthol-chloride (Cl 12251; basic brown no. 17), 2-((4-aminophenyl)azo)-1,3-dimethyl-1h-imidazol-3-ium-chloride (basic orange no. 31), 3,7-diamino-2,8-dimethyl-5-phenylphenazinium-chloride (CI 50240; basic red no. 2), 1,4-dimethyl-5-[(4-(dimethylamino)phenyl)azo]-1,2,4-triazolium-chloride (CI 11055; basic red no. 22), 1,3-dimethyl-2-((4-dimethylamino)phenyl)azo-1h-imidazol-3-ium-chloride (basic red no. 51), 2-hydroxy-1-[(2-methoxyphenyl)azo]-7-(trimethylammonio)-naphthalin-chloride (Cl 12245; basic red no. 76), 2-[2-((2,4-dimethoxyphenyl)amino)ethenyl]-1,3,3-trimethyl-3h-indol-1-ium-chloride (CI 48055; basic yellow no. 11), 3-methyl-1-phenyl-4-[(3-(trimethylammonio)phenyl)azo]-pyrazol-5-on-chloride (Cl 12719; basic yellow no. 57), di[4-(dimethylamino)phenyl]iminomethan-hydrochloride (CI 41000; basic yellow no. 2), 1-methyl-4-((methyl-phenylhydrazono)-methyl)-pyridinium-methylsulfate (basic yellow no. 87), bis[4-(diethylamino)phenyl]phenylcarbenium-hydrogensulfate (1:1) (CI 42040; basic green no.1), di(4-(dimethylamino)phenyl)-phenylmethanol (Cl 42000; basic green no. 4), 1-(2-morpholiniumpropylamino)-4-hydroxy-9,10-anthraquinone-methylsulfate, 1-[(3-(dimethylpropylaminium)propyl)-amino]-4-(methylamino)-9,10-anthraquinone-chloride, 1-[di(2-hydroxyethyl)amino]-3-methyl-4-[(4-nitrophenyl)azo]benzene (Cl 11210, disperse red no. 17), 1-[di(2-hydroxyethyl)amino]-4-[(4-nitrophenyl)azo]benzene (disperse black no. 9), 4-[(4-aminophenyl)azo]-1-[di(2-hydroxyethyl)amino]-3-methylbenzene (HC yellow no. 7), 2,6-diamino-3-[(pyridin-3-yl)azo]pyridine, 2-((4-(acetylamino)phenyl)azo)-4-methylphenol (Cl 11855; disperse yellow no. 3), 2-((4-(ethyl(2-hydroxyethyl)amino)-2-methylphenyl)azo)-5-nitro-1,3-thiazole (Cl 111935; disperse blue no. 106), 6-hydroxy-5-[(4-sulfophenyl)azo]-2-naphthalinesulfonic acid disodium salt (CI 15985; food yellow no. 3; FD&C yellow no. 6), 2,4-dinitro-1-naphthol-7-sulfonic acid disodium salt (Cl 10316; acid yellow no. 1; food yellow no. 1), 2-(indan-1,3-dion-2-yl)quinoline-x,x-sulfonic acid (mixture of mono- und disulfonic acid) (CI 47005; D&C yellow no. 10; food yellow no. 13, acid yellow no. 3), 5-hydroxy-1-(4-sulfophenyl)-4-[(4-sulfophenyl)azo]pyrazole-3-carboxylic acid trisodium salt (CI 19140; food yellow no. 4; acid yellow no. 23), 9-(2-carboxyphenyl)-6-hydroxy-3h-xanthen-3-one (CI 45350; acid yellow no. 73; D&C yellow no. 8), 4-((4-amino-3-sulfophenyl)azo)benzenesulfonic acid disodium salt (CI 13015, acid yellow no. 9), 5-[(2,4-dinitrophenyl)-amino]-2-phenylamino-benzenesulfonic acid sodium salt (CI 10385; acid orange no. 3), 4-[(2,4-dihydroxyphenyl)azo]-benzenesulfonic acid monosodium salt (CI14270; acid orange no. 6), 4-[(2-hydroxynaphth-1-yl)azo]-benzenesulfonic acid sodium salt (Ci 15510; acid orange no. 7), 4-((2-hydroxy-naphthalin-1-yl)azo)-3-methyl-benzenesulfonic acid sodium salt (CI 15575; acid orange no. 8), 4-[(2,4-dihydroxy-3-[(2,4-dimethylphenyl)azo]phenyl)azo]-benzenesulfonic acid sodium salt (CI 20170; acid orange no. 24), 3',6'-dihydroxy-4',5'-diiodospiro-(isobenzofuran-1(3h)-9'-(9h)xanthen)-3-one (Cl 45425, D&C orange no. 10), 4-hydroxy-3-[(4-sulfonaphth-1-yl)azo]-1-naphthalinesulfonic acid disodium salt (CI 14720; acid red no. 14), 4-hydroxy-3-[(2-methoxyphenyl)azo]-1-naphthalinesulfonic acid monosodium salt (CI 14710; acid red no. 4), 6-hydroxy-5-[(4-sulfonaphth-1-yl)azo]-2,4-naphthalinedisulfonic acid trisodium salt (Cl 16255; ponceau 4r; acid red no. 18), 3-hydroxy-4-[(4-sulfonaphth-1-yl)azo]-2,7-naphthalinedisulfonic acid trisodium salt (CI 16185; acid red no. 27), 8-amino-1-hydroxy-2-(phenylazo)-3,6-naphthalinedisulfonic acid disodium salt (CI 17200; acid red no. 33), 5-(acetylamino)-4-hydroxy-3-[(2-methylphenyl)azo]-2,7-naphthalinedisulfonic acid disodium salt (CI 18065; acid red no. 35), 2-(3-hydroxy-2,4,5,7-tetraiodo-dibenzopyran-6-on-9-yl)-benzoic acid disodium salt (ci45430;acid red no. 51), n-[6-(diethylamino)-9-(2,4-disulfophenyl)-3h-xanthen-3-ylidene]-n-ethylethanammoniumhydroxide, inner salt, sodium salt (CI 45100; acid red no. 52), 8-[(4-(phenylazo)-phenyl)azo]-7-naphthol-1,3-disulfonic acid disodium salt (CI 27290; acid red no. 73), 2',4',5',7'-tetrabromo-3',6'-dihydroxyspiro[isobenzofuran-1 (3h),9'-[9h]xanthen]-3-one disodium salt (CI 45380; acid red no. 87), 2',4',5',7'-tetrabromo-4,5,6,7-tetrachloro-3',6'-dihydroxyspiro-[isobenzofuran-1(3h),9'[9h]xanthen]-3-one disodium salt (Cl 45410; acid red no. 92), 3',6'-dihydroxy-4',5'-diiodospiro[isobenzofuran-1 (3h),9'(9h)-xanthen]-3-one disodium salt (CI 45425; acid red no. 95), 2-hydroxy-3-((2-hydroxynaphth-1-yl)azo)-5-nitrobenzenesulfonic acid monosodium salt (CI 15685; acid red no. 184), (2-sulfophenyl)di[4-(ethyl((4-sulfophenyl)methyl)-amino)phenyl]carbenium disodium salt, betaine (CI 42090; acid blue no. 9; FD&C blue no. 1), 3-hydroxy-4-((4-methyl-2-sulfophenyl)azo)-2-naphthalinecarboxylic acid disodium salt (Cl 15850; d&c red no. 6), 6-hydroxy-5-((2-methoxy-5-methyl-4-sulfophenyl)azo)-2-naphthaline-sulfonic acid disodium salt (Cl 16035; FD&C red 40), 1,4-bis[(2-sulfo-4-methylphenyl)amino]-9,10-anthraquinone disodium salt (Cl 61570; acid green no. 25), bis[4-(dimethylamino)phenyl]-(3,7-disulfo-2-hydroxynaphth-1-yl) carbenium inner salt, mononsodium salt (Cl 44090; food green no. 4; acid green no. 50), bis[4-(diethylamino)phenyl](2,4-disulfophenyl)-carbenium inner salt, sodium salt (2:1) (Cl 42045; food blue no. 3; acid blue no. 1), bis[4-(diethylamino)phenyl](5-hydroxy-2,4-disulfophenyl)-carbenium inner salt, calcium salt (2:1) (CI 42051; acid blue no. 3), 1-amino-4-(cyclohexylamino)-9,10-anthraquinone-2-sulfonic acid sodium salt (CI 62045; acid blue no. 62), 3,3-bis(3,5-dibromo-4-hydroxyphenyl)-4,5,6,7-tetrabromo-2,1(3h)-benzoxathiol-1,1-dioxide, 1-amino-4-(phenylamino)-9,10-anthraquinone-2-sulfonic acid (Cl 62055; acid blue no. 25), 2-(1,3-dihydro-3-oxo-5-sulfo-2h-indol-2-ylidene)-2,3-dihydro-3-oxo-1h-indol-5-sulfonic acid disodium salt (CI 73015; acid blue no. 74), 9-(2-carboxyphenyl)-3-[(2-methylphenyl)amino]-6-[(2-methyl-4-sulfophenyl)amino]xanthylium inner salt, mononsodium salt (Cl 45190; acid violet no. 9), 1-hydroxy-4-[(4-methyl-2-sulfophenyl)amino]-9,10-anthraquinone sodium salt (Cl 60730; d&c violet no. 2; acid violet no. 43), bis[3-nitro-4-[(4-phenylamino)-3-sulfo-phenylamino]phenyl]sulfone (Cl 10410; acid brown no. 13), 5-amino-4-hydroxy-6-[(4-nitrophenyl)azo]-3-(phenylazo)-2,7-naphthaline disulfonic acid disodium salt (Cl 20470; acid black no. 1), 3-hydroxy-4-[(2-hydroxynaphth-1-yl)azo]-7-nitro-1-naphthalinesulfonic acid chromium complex (3:2) (Cl15711; acid black no. 52), 3-[(2,4-dimethyl-5-sulfophenyl)azo]-4-hydroxy-1-naphthalinesulfonic acid disodium salt (Cl 14700; food red no. 1; ponceau sx; FD&C red no. 4), 4-(acetylamino)-5-hydroxy-6-[(7-sulfo-4-[(4-sulfophenyl)azo]naphth-1-yl)azo]-1,7-naphthalinedisulfonic acid tetrasodium salt (Cl 28440; food black no. 1), 3-hydroxy-4-(3-methyl-5-oxo-1-phenyl-4,5-dihydro-1h-pyrazole-4-ylazo)-naphthaline-1-sulfonic acid sodium salt, chromium complex (acid red no. 195).

The direct dyes and/or oxidation dyes/dye precursors are contained in the ready-to-use agent in a total amount from about 0.01 to 20 weight percent, preferably from about 0.1 to 10 weight percent, and most preferably from about 0.1 to 5 weight percent.

The agents according to the present invention also comprise at least one source of an oxidizing agent. Preferred oxidizing agents for use herein are water-soluble peroxygen oxidizing agents. "Water-soluble" as defined herein means that in standard condition at least 0.1 g, preferably 1 g, more preferably 10 g of said oxidizing agent can be dissolved in 1 liter of deionized water. The oxidizing agents are valuable for the initial solubilization and decolorization of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulfates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is dissolved prior to use. Preferred for use in the agents according to the present invention are hydrogen peroxide, percarbonates, persulfates and combinations thereof.

According to the present invention the agents comprise from about 0.1 % to about 15% by weight, preferably from about 1% to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 10.5, preferably 7.5 to 10.5 more preferably about pH 10. Moreover, this system is also particularly effective in combination with a source of ammonia or ammonium ions. It has been found that this oxidizing agent can deliver improvements to the desired hair color results particularly with regard to the delivery of high lift, whilst considerably reducing the odor, skin and scalp irritation and damage to the hair fibers.

Accordingly, any source of these ions may be utilized. Suitable sources for use herein include sodium, potassium, guanidine, arginine, lithium, calcium, magnesium, barium, ammonium salts of carbonate, carbamate and hydrogencarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the agents may contain from about 0.1 % to about 15% by weight, preferably from about 1% to about 10% by weight, and most preferably from about 1 % to about 8% by weight of a hydrogencarbonate ion and from about 0.1 % to about 10% by weight, preferably from about 1 % to about 7% by weight, and most preferably from about 2% to about 5% by weight of a source of hydrogen peroxide.

According to the present invention the agent may further optionally comprise at least one source of alkalizing agent, preferably a source of ammonium ions and or ammonia. Any agent known in the art may be used such as alkanolamides for example monoethanolamine, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts. Particularly preferred alkalizing agents are those which provide a source of ammonium ions. Any source of ammonium ions is suitable for use herein. Preferred sources include ammonium chloride, ammonium sulfate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium carbamate, ammonium hydrogen carbonate, ammonia and mixtures thereof. The agents of the present invention may comprise from about 0.1 % to about 10% by weight, preferably from about 0.5% to about 5%, most preferably from about 1% to about 3% of an alkalizing agent, preferably ammonium ions.

The agent according to the invention may also contain one or more additive commonly used in solutions, creams, emulsions, gels or aerosol foams, powders and granulates, for example solvents, such as water, low aliphatic monohydroxy or polyhydroxy alcohols, their esters and ethers, for example alkanols, particularly with an alkyl chain comprising 1 to 4 carbon atoms, such as ethanol, n-propanol or isopropanol, butanol, isobutanol; bivalent or trivalent alcohols, particularly of the type having 2 to 6 carbon atoms, such as ethylene glycol, propylene glycol, 1,3-propanediol, 1,4-butanediol, 1,5-pentanediol, 1,6-hexanediol, 1,2,6-hexanetriol, glycerine, diethylene glycol, dipropylene glycol, polyalkylene glycols, such as triethylene glycol, polyethylene glycol, tripropylene glycol and polypropylene glycol; low alkyl ethers of multivalent alcohols, such as ethylene glycol monomethyl ether, ethylene glycol monoethyl ether, ethylene glycol monopropyl ether or ethylene glycol monobutyl ether, diethylene glycol monomethyl ether or diethylene glycol mono ethyl ether, triethylene glycol monomethyl ether or triethylene glycol mono ethyl ether; ketones and keto alcohols, especially such with 3 to 7 carbon atoms in their molecules, such as acetone, methyl ethyl ketone, diethyl ketone, methyl isobutyl ketone, methyl phenyl ketone, cyclopentanone, cyclo hexanone and diacetone alcohol; ethers such as dibutyl ether, tetra-hydrofurane, dioxane or diisopropyl ether; esters such as ethyl formate, methyl formate, methyl acetate, ethyl acetate, propyl acetate, butyl acetate, phenyl acetate, ethylene glycol monoethyl ether acetate or acetic acid hydroxy ethyl ester; amids such as dimethyl formamide, dimethyl acetamide or N-methyl-pyrrolidone; as well as urea, tetramethyl urea and thiodiglycol; moreover, wetting agents or emulsifyers from the group of anionic, cationic, non-ionogenic, amphoteric or zwitterionic surface-active substances, such as fatty aclohol sulfates, alkyl-sulfonates, alkylbenzene sulfonates, alkyltrimethyl ammonium salts, alkylbetaines, α-olefin sulfonates, oxethylated fatty alcohols, oxethylated nonylphenoles, fatty acid alkanolamines, oxethylated fatty acid esters, fatty alcohol polyglycol ether sulfates, alkylpolyglucosides; thickening agents, such as high fatty alcohols, starch, cellulose derivatives, vaseline, paraffin oil, fatty acids and other fatty components in emulsified form, water-soluble polymeric thickening agents, for instance natural rubbers, guar gum, xanthan gum, carob flour, pectine, dextrane, agar-agar, amylose, amylopectine, dextrine, clays or synthetic hydrocolloides, such as polyvinyl alcohol; also conditioning agents, such as lanolin derivatives, cholesterol, pantothenic acid, water-soluble cationic polymers, protein derivatives, provitamins, vitamins, plant extracts, sugar and betaine; auxiliary agents, such as electrolytes, antioxidants, fatty amides, sequestrants, film-forming agents and preservatives.

The aforementioned compounds are used in the amounts usual for such purposes, for example, wetting agents and emulsifying agents in concentrations of about 0.5 to 30 % by weight, thickening agents of about 0.1 to 25 % by weight, conditioning agents in concentrations of about 0.1 to 5.0 % by weight.

The oxidizing agent and the alkalizer of the coloring agent according to the present invention normally must be kept separately during storage and will be mixed direct prior to use. The agents according to the present invention may be packed in different ways. For instance the agents are in the form of a multi-component, preferably a 2-component-kit consisting of a component (A) containing the oxidizing agent and (B) containing at least one bleach booster of formula (I), at least one direct dye and/or at least one oxidation dye/dye precursor and if needed an agent for adjusting the pH. In another preferred embodiment the 2-component-kit consists of a component (A) containing at least one bleach booster of formula (I) and at least one oxidizing agent and a component (B) containing at least one direct dye and/or at least one oxidation dye/dye precursor and if needed an agent for adjusting the pH.

The pH of the ready-to-use agent is preferably from about 2 to about 12, more preferably from about 4 to about 11, and most preferably from about 6 to about 10,5.

The components are mixed prior to the application and the ready-to-use agent is spread on the fibers to be colored, if needed by addition of water or an aqueous solution containing usual cosmetic additives. The mixture is left on the fibers, such as hairs, at about 10 to 70 °C, preferably at about 20 to 50 °C, for about 5 to 60 minutes, preferably for about 30 and 50 minutes, then the fibers are rinsed with water, optionally washed with a shampoo and/or a conditioner, rinsed with water and dried.

The coloring method according to the present invention achieves (especially on human hair) color results with excellent fastness properties, especially with regard to light, shampooing and friction. The color results thus achieved are, irrespective of different hair structures, uniform and highly reproducible.

The following examples further illustrate the invention in more detail, but they should not be construed as limiting the appended claims.

### Examples

### Example 1: Oxidative Hair Coloring Agent

### Component (A1)

- cetearyl alcohol: 2,400 g
- sodium lauryl sulfate: 0,250 g
- disodium phosphate: 0,200 g
- phosphoric acid (85 % aqueous solution): 0,150 g
- salicylic acid: 0,100 g
- etidronic acid (60 % aqueous solution): 0,001 g
- sodium cyclohexanesulfamate: 23,00 g
- hydrogen peroxide (50 % aqueous solution): 9,350 g
- water, demineralized: to 100.000 g

The ingredients are heated up to 50 °C and cooled down to room temperature.

### Component (A2)

- cetearyl alcohol: 2,400 g
- sodium lauryl sulfate: 0,250 g
- 1-hydroxyethyl 4,5-diamono pyrazole sulfate: 8,500 g
- 4-amino-2-hydroxytoluene: 5,000 g
- ammonia (25 % aqueous solution): 23,00 g
- disodium ethylenediaminetetraacetate hydrate: 0,200 g
- water, demineralized: to 100.000 g
9 parts of component (A1) are mixed with 1 part of component (A2). The pH of the ready-to-use colorant is at about 10. The agent is applied to medium brown natural hair and uniformly distributed with a brush. After an exposure time of 30 minutes at 40 °C, the hair is washed with a commercial shampoo, rinsed with lukewarm water, treated with a commercial conditioner, rinsed with water again and then dried. The hair shows a brilliant orange color.
[A reference hair strand treated with a mixture of 9 parts of component (A1), containing water instead of the bleach booster (sodium cyclohexanesulfamate), and 1 part of component (A2) is colored orange brown.]

### Example 2: Hair Coloring Agent with Direct dyes

### Component (A1)

- cetearyl alcohol: 2,400 g
- sodium lauryl sulfate: 0,250 g
- disodium phosphate: 0,200 g
- phosphoric acid (85 % aqueous solution): 0,150 g
- salicylic acid: 0,100 g
- etidronic acid (60 % aqueous solution): 0,001 g
- sodium cyclohexanesulfamate: 19,600 g
- hydrogen peroxide (50 % aqueous solution): 6,600 g
- water, demineralized: to 100.000 g

The ingredients are heated up to 50 °C and cooled down to room temperature.

### Component (A2)

- cetearyl alcohol: 2,400 g
- sodium lauryl sulfate: 0,250 g
- 4-amino-3-nitrophenol: 5,000 g
- 2-amino 6-chlor 4-nitrophenol: 5,000 g
- ammonia (25 % aqueous solution): 30,000 g
- water, demineralized: to 100.000 g
9 parts of component (A1) are mixed with 1 part of component (A2). The pH of the ready-to-use colorant is at about 10. The agent is applied to medium brown natural hair and uniformly distributed with a brush. After an exposure time of 30 minutes at 40 °C, the hair is washed with a commercial shampoo, rinsed with lukewarm water, treated with a commercial conditioner, rinsed again with water and then dried. The hair shows a brilliant orange color.
[A reference hair strand treated with a mixture of 9 parts of component (A1), containing water instead of the bleach booster (sodium cyclohexanesulfamate), and 1 part of component (A2) is colored orange brown.]

### Example 3-13: Hair Coloring Agent with Direct dyes

- direct dye(s) (see table 1): 1,000 g
- sodium cyclohexanesulfamate: 17,500 g
- hydrogen peroxide (50 % aqueous solution): 6,000 g
- ammonia (25 % aqueous solution): 4,600 g
- water, demineralized: to 100.000 g

The direct dyes are mixed with water, the sodium cyclohexanesulfamate and the hydrogen peroxide and heated up to 50 °C and cooled down to room temperature. Then the ammonia is added. The pH of the ready-to-use colorant is at about 10. The agent is applied to medium brown natural hair and uniformly distributed with a brush. After an exposure time of 30 minutes at 40 °C, the hair is washed with a commercial shampoo, rinsed with lukewarm water, treated with a commercial conditioner, again rinsed with water and then dried. The coloration results on middle brown hair are always compared to an equal formulation but with water instead of the bleach booster (sodium cyclohexanesulfamate).

**Tabelle 1:**

| **No.** | **dye(s)** | **coloration result with bleach booster** | **coloration result with water instead of the bleach booster** |
|---|---|---|---|
| 3 | 4-amino-3-nitrophenol | brilliant orange | dull orange |
| 4 | 3-nitro-p-hydroxyethylamino-phenol | brilliant red | dull red |
| 5 | 4-nitrophenylaminoethylurea | light yellow brown | middle yellow brown |
| 6 | 2,6-diamino-3-((pyridin-3-yl)azo)pyridine | light warm yellow brown | middle warm yellow brown |
| 7 | hydroxyethyl-2-nitro-p-toluidine | brilliant orange | dull orange |
| 8 | 2-chloro-6-ethylamino-4-nitrophenol | brilliant red | dull red |
| 9 | Acid Yellow No. 1 | light yellow brown | middle yellow brown |
| 10 | Acid Red No. 92 | brilliant pink | dull pink |
| 11 | Acid Red No. 27 | light pink brown | dull pink brown |
| 12 | Basic Red No. 51 | light brilliant red | dull red |
| 13 | 4-amino-3-nitrophenol 0,5 g; 3-nitro-p-hydroxyethylamino-phenol 0,5 g | brilliant orange red | dull orange red |

### Example 14: Oxidative Hair Coloring Agent

### Component (A1)

- cetearyl alcohol: 2,400 g
- sodium lauryl sulfate: 0,250 g
- disodium phosphate: 0,200 g
- phosphoric acid (85 % aqueous solution): 0,150 g
- salicylic acid: 0,100 g
- etidronic acid (60 % aqueous solution): 0,001 g
- sodium cyclohexanesulfamate: 23,00 g
- hydrogen peroxide (50 % aqueous solution): 9,350 g
- water, demineralized: to 100.000 g

The ingredients are heated up to 50 °C and cooled down to room temperature.

### Component (A2)

- cetearyl alcohol: 2,400 g
- sodium lauryl sulfate: 0,250 g
- 1-hydroxyethyl 4,5-diamono pyrazole sulfate: 8,500 g
- 4-amino-2-hydroxytoluene: 5,000 g
- 3-nitro-p-hydroxyethylaminophenol: 5,000 g
- ammonia (25 % aqueous solution): 23,00 g
- disodium ethylenediaminetetraacetate hydrate: 0,200 g
- water, demineralized: to 100.000 g
9 parts of component (A1) are mixed with 1 part of component (A2). The pH of the ready-to-use colorant is at about 10. The agent is applied to medium brown strands of natural hair and uniformly distributed with a brush. After an exposure time of 30 minutes at 40 °C, the hair is washed with a commercial shampoo, rinsed with lukewarm water, treated with a commercial conditioner, rinsed again with water and then dried. The hair shows a brilliant orange red color.
[A reference hair strand treated with a mixture of 9 parts of component (A1), but with water instead of the bleach booster (sodium cyclohexanesulfamate), and 1 part of component (A2) is colored orange red brown.]

### Example 15: Agent for Simultaneously Lightening and Coloring Hair (Direct Dye)

- glycerin (86 % aqueous solution): 15,000 g
- potassium persulfate: 7,500 g
- sodium cyclohexanesulfamate: 23,000 g
- hydrogen peroxide (50 % aqueous solution): 9,000 g
- Acid Red No. 92: 1,000 g
- water, demineralized: to 100.000 g

The ingredients (apart from the potassium persulfate) are heated up to 50 °C and cooled down to room temperature. Then the potassium persulfate is added.

The pH is adjusted with ammonia (25 % aqueous solution) to 10,2.

The agent is applied to medium brown natural hair and uniformly distributed with a brush. After an exposure time of 30 minutes at 40 °C, the hair is washed with a commercial shampoo, rinsed with lukewarm water, treated with a commercial conditioner, rinsed again with water and then dried. The hair shows a brilliant pink color.
[A reference hair strand treated with the same agent but containing water instead of the bleach booster (sodium cyclohexanesulfamate), is brownish pink colored.]

### Example 16: Lightening Oxidative Dye Agent

- glycerin (86 % aqueous solution): 15,000 g
- potassium persulfate: 5,000 g
- sodium cyclohexanesulfamate: 23,000 g
- hydrogen peroxide (50 % aqueous solution): 9,000 g
- disodium ethylenediaminetetraacetate hydrate: 0,300 g
- 1-hydroxyethyl 4,5-diamono pyrazole sulfate: 1,200 g
- 4-amino-2-hydroxytoluene: 0,620 g
- water, demineralized: to 100.000 g

The ingredients (apart from the potasium persulfate) are heated up to 50 °C and cooled down to room temperature. Then the potassium persulfate is added.

The pH is adjusted with ammonia (25 % aqueous solution) to 10.

The agent is applied to medium brown natural hair and uniformly distributed with a brush. After an exposure time of 30 minutes at 40 °C, the hair is washed with a commercial shampoo, rinsed with lukewarm water, treated with a commercial conditioner, rinsed again with water and then dried. The hair shows a brilliant orange color.
[A reference hair strand treated with the same agent but containing water instead of the bleach booster, is brownish orange colored.]

### Example 17: Agent for Simultaneously Lightening and Coloring Hair (Direct Dye)

- ethanol: 10,000 g
- laureth-4: 10,000 g
- sodium persulfate: 2,500 g
- sodium cyclohexanesulfamate: 30,000 g
- Polyquaternium-7 (8 % in water): 3,000 g
- hydrogen peroxide (50 % aqueous solution): 12,000 g
- Acid Red No. 92: 0,500 g
- hydroxyethyl-2-nitro-p-toluidine: 0,500 g
- water, demineralized: to 100.000 g

The ingredients (apart from the sodium persulfate) are heated up to 50 °C and cooled down to room temperature. Then the sodium persulfate is added.

The pH is adjusted with ammonia (25 % aqueous solution) to 10,2.

The agent is applied to medium brown natural hair and uniformly distributed with a brush. After an exposure time of 30 minutes at 40 °C, the hair is washed with a commercial shampoo, rinsed with lukewarm water, treated with a commercial conditioner, rinsed again with water and then dried. The hair shows a brilliant red color.
[A reference hair strand treated with the same agent but containing water instead of the bleach booster (sodium cyclohexanesulfamate), is brownish red colored.]

Unless otherwise indicated, all percentages in the present patent application are percentages by weight.

## Claims

1. A ready-to-use agent for simultaneously lightening and dyeing keratin fibers (especially human hair), having a basic pH and being **characterized by** containing
a) at least one bleach booster according to the general formula **(I)** 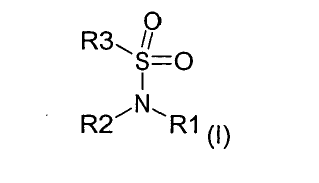 wherein **R1** is a hydrogen, **R2** is hydrogen, a substituted or unsubstituted C1- to C12-alkyl group, a substituted or unsubstituted C1- to C12-monohydroxy-alkyl group, a substituted or unsubstituted C2- to C12-polyhydroxy alkyl group, or a substituted saturated, unsaturated or aromatic 4- to 8-membered carbocycle or heterocycle, and **R3** is a OR-Group, with **R** being equal to hydrogen, an ammonium group or an alkali metal or an earth alkali metal atom; and
b) at least one direct dye and/or at least one oxidation dye/dye precursor and c) at least one oxidant.

2. Agent according to claim 1, **characterized in that** the bleach booster of the general formula (I) is selected from the group consisting of sodium cyclohexanesulfamate; ammonium cyclohexanesulfamate; potassium cyclohexanesulfamate; cyclohexane sulfamic acid; sulfamic acid; sodium sulfamate; potassium sulfamate; ammonium sulfamate; sodium phenylsulfamate; ammonium phenylsulfamate; potassium phenylsulfamate; phenyl sulfamic acid; sodium benzylsulfamate; ammonium benzylsulfamate; potassium benzylsulfamate; benzyl sulfamic acid; sodium isopropylsulfamate; ammonium isopropylsulfamate; potassium isopropylsulfamate; isopropyl sulfamic acid; sodium methylsulfamate; ammonium methylsulfamate; potassium methylsulfamate; methyl sulfamic acid; sodium t-butylsulfamate; ammonium t-butylsulfamate; potassium t-butylsulfamate and t-butyl sulfamic acid.

3. Agent according to claim 1 or 2, **characterized in that** the bleach booster is contained in an amount from 0.01 to 70 weight percent.

4. Agent according to one of claims 1 to 3, **characterized in that** the direct dyes and/or oxidation dyes/dye precursors are contained in the ready-to-use agent in a total amount from 0.01 to 20 weight percent.

5. Agent according to one of claims 1 to 4, **characterized in that** the oxidizing agent is selected from the group consisting of hydrogen peroxide, inorganic alkali metal peroxides, organic peroxides, inorganic perhydrate salt bleaching compounds, monohydrates or tetrahydrates of inorganic perhydrate salt bleaching compounds, alkyl peroxides, aryl peroxides, peroxidases and mixtures of these compounds.

6. Agent according to one of claims 1 to 5, **characterized in that** the oxidizing agent is selected from the group consisting of hydrogen peroxide, percarbonates, persulfates and combinations thereof.

7. Agent according to one of claims 1 to 6, **characterized in that** the oxidizing agent is contained in a total amount of from 0.1 to 15 weight percent.

8. Agent according to one of claims 1 to 7, **characterized in that** its pH is from 2 to 12.

9. Multicomponent kit consisting of a component (A) containing at least one compound of formula (I) and at least one oxidizing agent, and a component (B) containing at least one direct dye and/or at least one oxidation dye/dye precursor, and if needed an agent for adjusting the pH.

10. Multicomponent kit consisting of a component (A) containing at least one oxidizing agent, and a component (B) containing at least one compound of formula (I) and at least one direct dye and/or at least one oxidation dye/dye precursor, and if needed an agent for adjusting the pH.

11. Method for the simultaneous lightening and coloring of hair comprising the steps of (i) applying an agent according to one of claims 1 to 8 to the hair, (ii) after an exposure time of 5 to 60 minutes at a temperature of 10 to 70 °C rinsing the hair with water, (iii) optionally washing the hair with a shampoo and/or a conditioner, (iv) rinsing the hair with water and (v) finally drying the hair.
